# EUROPEAN PATENT SPECIFICATION

(11) **EP 4 522 584 B1**
(45) Date of publication and mention of the grant of the patent: **22.10.2025**
(21) Application number: 23719863.5
(22) Date of filing: 12.04.2023
(51) Int. Cl.: C07C 29/80, C07C 29/151, C07C 31/04, C01B 3/02, C01B 3/38

(54) **PROCESS FOR SYNTHESISING METHANOL**
VERFAHREN ZUR SYNTHESE VON METHANOL
PROCÉDÉ DE SYNTHÈSE DE MÉTHANOL

(30) Priority: 09.05.2022 GB 202206756
(43) Date of publication of application: 19.03.2025
(73) Proprietor: Johnson Matthey Davy Technologies Limited, London EC2V 7AD (GB)
(72) Inventor: CASSIDY, Paul John, London W2 6LG (GB); YORATH, Neil David, London W2 6LG (GB)
(74) Representative: Johnson Matthey Plc
(86) International application number: PCT/GB2023/050982
(87) International publication number: WO 2023/218160

(56) References cited:
- GB-A- 2 585 477
- US-B2- 9 422 212

## Description

This invention relates to a process for synthesising methanol, in particular a process for synthesising methanol with low emissions of carbon dioxide from the process.

Methanol synthesis is generally performed by passing a synthesis gas comprising hydrogen and carbon monoxide and/or carbon dioxide at an elevated temperature and pressure through one or more beds of a methanol synthesis catalyst, which is often a copper-containing composition, in a synthesis reactor. A crude methanol is generally recovered by cooling the product gas stream to below the dew point and separating off the product as a liquid. The crude methanol is typically purified by distillation. The process is often operated in a loop: thus unreacted gas may be recycled to the synthesis reactor as part of the feed gas via a circulator. Fresh synthesis gas, termed make-up gas, is added to the recycled unreacted gas to form the feed gas stream. A purge stream is taken from the circulating gas stream to avoid the build-up of inert gasses in the loop.

Various methods exist to recover hydrogen from the purge gas and re-use it in the methanol synthesis step, especially for synthesis gases that are hydrogen deficient. After such hydrogen separation, the resulting tail gas is typically used as a fuel combusted to heat feeds for the process, or in the generation of steam. For example, GB2585477A describes a methanol synthesis process in which a purge gas stream is separated into a hydrogen-rich stream and a carbon-rich stream, a portion of the hydrogen-rich stream is fed to the methanol synthesis loop and a portion of the carbon-rich stream is fed to the reforming unit. A fraction of the carbon-rich stream may be burned as fuel. Because the tail gas contains carbon-containing compounds, such as methane, the combustion of the purge gas leads to CO₂ emissions from the process.

Processes, where this carbon is re-used in the process are known. For example, WO2020/249924 A1 discloses a methanol synthesis process utilising a reforming unit comprising a heat exchange reformer and an autothermal reformer in series for the synthesis gas generation, wherein a hydrogen-rich stream and a carbon-rich stream are separated from the purge gas stream, a portion of the hydrogen-rich stream is fed to the methanol synthesis loop and a portion of the carbon-rich stream is fed to the reforming unit.

The Applicant has found that, rather than use the purge gas as a source of hydrogen for the methanol synthesis, it may advantageously be used in reforming units comprising a fired steam reformer as fuel.

Accordingly the invention provides a process for synthesising methanol comprising the steps of (i) reforming a hydrocarbon feedstock in a hydrocarbon reforming unit comprising a fired steam reformer to form a synthesis gas containing hydrogen, carbon monoxide and carbon dioxide; (ii) converting the synthesis gas into a methanol product in a methanol loop comprising one or more methanol synthesis reactors; and (iii) recovering a purge gas stream from the methanol loop, wherein at least a portion of the purge gas stream is treated in a purge gas treatment unit by

subjecting it to partial oxidation in a partial oxidation reactor or reforming in a purge gas reforming unit to form a partially-oxidised or reformed purge gas, followed by one or more stages of water gas shift of the partially-oxidised or reformed purge gas in a water-gas shift unit to form a hydrogen-enriched gas, and a step of carbon dioxide removal from the hydrogen-enriched gas in a carbon dioxide removal unit to form a hydrogen stream and a carbon dioxide stream, wherein the carbon dioxide stream is recovered and a portion of the hydrogen stream is fed to the fired steam reformer as a fuel.

This process may be established in a new methanol production unit, or an existing methanol production unit may be retrofitted with a partial oxidation reactor or a purge gas reforming unit, a water-gas shift unit and a carbon dioxide removal unit and means to recover carbon dioxide and feed a hydrogen stream recovered from the carbon dioxide removal unit to the fired steam reformer as a fuel.

Accordingly, the invention further provides a method for retrofitting a methanol production unit comprising a hydrocarbon reforming unit comprising a fired steam reformer and a methanol loop comprising one or more methanol synthesis reactors, wherein the methanol loop is fed with a synthesis gas from the hydrocarbon reforming unit and generates a methanol product and a purge gas stream, by (i) installing a purge gas treatment unit comprising a partial oxidation reactor or a purge gas reforming unit, a water-gas shift unit, a carbon dioxide removal unit and means to feed a hydrogen stream recovered from the carbon dioxide removal unit to the fired steam reformer as a fuel, (ii) passing at least a portion of the purge gas stream to the partial oxidation reactor or purge gas reforming unit to form a partially-oxidised or reformed purge gas stream, (iii) subjecting the partially-oxidised or reformed purge gas stream to one or more stages of water gas shift in the water-gas shift unit to form a hydrogen-enriched stream, (iv) subjecting the hydrogen-enriched stream to carbon dioxide removal in the carbon dioxide removal unit to form a hydrogen stream, and (v) feeding the hydrogen stream to the fired steam reformer as a fuel.

Together, the partial oxidation reactor or purge gas reforming unit, the water gas shift unit and the carbon dioxide removal unit may be described as a purge gas treatment unit. The use or installation of a purge gas treatment unit according to the present invention offer operators a means to significantly decarbonise the methanol process by replacing a carbon containing fuel with a hydrogen fuel for the fired steam reformer. The hydrogen fuel may also be used in place of natural gas in any fired heaters used in the process or the purge gas treatment unit to preheat feeds or generate steam for the process.

The hydrocarbon feed may be any gaseous or low boiling hydrocarbon, such as natural gas, associated gas, LPG, petroleum distillate, diesel, naphtha or mixtures thereof, or hydrocarbon-containing off-gases from chemical processes, such as a refinery off-gas or a pre-reformed gas containing methane. The gaseous mixture preferably comprises methane, associated gas or natural gas containing a substantial proportion, e.g. over 50% by volume methane. Natural gas is especially preferred. The hydrocarbon may be compressed to a pressure in the range 10-100 bar abs. The pressure of the hydrocarbon may usefully govern the pressure throughout the process. Operating pressure is preferably in the range 15-50 bar abs, more preferably 25-50 bar abs as this provides an enhanced performance from the process.

If the hydrocarbon feedstock contains sulphur compounds, before or after compression, the feedstock is preferably subjected to desulphurisation, e.g. hydrodesulphurisation using Co or Ni catalysts and absorption of hydrogen sulphide using a suitable absorbent, e.g. a zinc oxide bed. To facilitate this hydrogen is preferably added to the hydrocarbon feedstock. The amount of hydrogen in the resulting mixed gas stream may be in the range 1-20% vol, but is preferably in the range 1-10%, more preferably in the range 1-5%. In a preferred embodiment a portion of the hydrogen stream is mixed with the hydrocarbon feed stream. The hydrogen stream may be combined with the hydrocarbon upstream of any hydrodesulphurisation stage. The resulting desulphurised hydrocarbon feedstock may also be fed to the purge gas treatment unit.

If the hydrocarbon feedstock contains other contaminants, such as chloride or heavy metal contaminants, these may be removed, prior to reforming, upstream or downstream of any desulphurisation, using conventional adsorbents. Adsorbents suitable for chloride removal are known and include alkalised alumina materials. Similarly, adsorbents for heavy metals such as mercury or arsenic are known and include copper sulphide materials.

The hydrocarbon feedstock is subjected to steam reforming in the hydrocarbon reforming unit. In steam reforming, the hydrocarbon feedstock is mixed with steam: this steam introduction may be effected by direct injection of steam and/or by saturation of the hydrocarbon feedstock by contact of the latter with a stream of heated water in a saturator. One or more saturators may be used. If desired, a portion of the hydrocarbon feedstock may bypass the steam addition, e.g. the saturator. The amount of steam introduced may be such as to give a steam to carbon ratio at the inlet to the fired steam reformer of 1.5 to 3, preferably about 2, i.e. 2 moles of steam per gram atom of hydrocarbon carbon in the hydrocarbon feedstock. The amount of steam is preferably minimised as this leads to a lower cost, more efficient process.

Where the hydrocarbon is a rich natural gas, naphtha or other hydrocarbon-containing feedstock containing hydrocarbons heavier than methane it may be desirable to subject it to a step of pre-reforming upstream of the fired steam reformer and/or the purge gas treatment unit. Pre-reforming processes are known. In such processes, the hydrocarbon/steam mixture is heated, typically to a temperature in the range 400 to 650°C, and then passed adiabatically through a fixed bed of a suitable particulate steam reforming catalyst, usually a precipitated catalyst having a high nickel content, for example above 40% by weight, expressed as NiO. During such an adiabatic reforming step, any hydrocarbons higher than methane react with steam to give a pre-reformed gas comprising a mixture of methane, carbon oxides and hydrogen. The use of pre-reforming step is desirable to ensure that the feed to the fired steam reformer contains no hydrocarbons higher than methane and also contains a significant amount of hydrogen. This is desirable in order to minimise the risk of carbon formation on the catalyst in the fired steam reformer.

The hydrocarbon/steam mixture is desirably pre-heated prior to reforming in the fired steam reformer. This may be achieved by passing the feed though heat exchange coils in a convection section of the fired steam reformer, and/or by using a fired heater. If a fired heater is used, then it is preferably heated by combustion of a portion of the hydrogen stream. Desirably, the mixed stream is heated to an inlet temperature in the range 300 to 650°C. Inlet temperatures in the range of 300 to 550°C are particularly suitable when there is no pre-reformer and higher inlet temperatures in the range of 550 to 650°C are particularly suitable when there is a pre-reformer.

The hydrocarbon feedstock/steam gas mixture is then subjected to reforming in the hydrocarbon reforming unit comprising a fired steam reformer, which may also be termed a fired steam reformer, or a fired catalytic steam reformer because steam is used to convert the hydrocarbon feedstock into synthesis gas over a catalyst. Fired steam reformers are known and generally comprise a radiant section containing a plurality of catalyst-containing reformer tubes through which the mixture of hydrocarbon feedstock and steam is passed. The reformer tubes are typically arranged vertically in rows. Fuel and air are fed to a plurality of burners in the walls of the radiant section of the fired steam reformer that combust the fuel to generate heat for the endothermic steam reforming reactions. The fired steam reformer may be a side-fired reformer or a top-fired reformer. The combustion gas is typically then conveyed through a downstream convection section of the fired steam reformer where it may be used to heat feed streams and /or generate steam, before being discharged as a flue gas.

During the reforming process, methane reacts with steam to produce a synthesis gas comprising hydrogen, carbon monoxide and carbon dioxide. Any hydrocarbons containing two or more carbon atoms that are present are converted to methane, carbon monoxide and hydrogen. In addition, water-gas shift reactions occur. Overall, the process is endothermic, requiring heating of the tubes and catalyst to maintain the reaction and achieve the desired conversion. The heat input to the steam reformer is typically such that the temperature of product gas stream at the outlet of the tubes is higher than the inlet temperature, often in the range of 100 to 350 degrees Celsius higher than the inlet temperature.

The fired steam reformer may be operated with a relatively high exit temperatures, e.g. above 850°C, such that the methane content of the synthesis gas is low, but in some arrangements the exit temperature may be lower, e.g. in the range 700-850°C, and in consequence the fuel demand reduced, where a secondary reformer is included in the hydrocarbon reforming unit downstream of the fired, or primary, reformer. The secondary reformer is preferably an autothermal reformer. Accordingly in some arrangements, the reforming unit comprises a fired steam reformer and an autothermal reformer. The reformed gas from the fired steam reformer is fed to the autothermal reformer to convert residual methane in the primary reformed gas into synthesis gas. In a preferred arrangement, the autothermal reformer may also be fed with a portion of the hydrocarbon feedstock to increase the synthesis gas production. The portion of the total hydrocarbon feedstock that is fed to the autothermal reformer may be in the range 5-60% by volume, or 60-95% by volume or 33-70% by volume. Such combined reforming is known and is described, for example, in US4888130. The combination of a portion of the hydrocarbon feedstock with the primary reformed gas provides a convenient method of producing a synthesis gas having an ideal stoichiometry for methanol synthesis. The combined feed to the autothermal reformer may have a steam to carbon ratio of 0.6 or higher, preferably 0.9 or higher.

The autothermal reformer will generally comprise a burner disposed near the top of the reformer, to which is fed the primary reformed gas mixture and an oxygen-containing gas, a combustion zone beneath the burner through which, typically, a flame extends, above a fixed bed of particulate steam reforming catalyst. In autothermal or secondary reforming, the heat for the endothermic steam reforming reactions is provided by combustion of hydrocarbon and hydrogen in the feed gas. The reformed gas mixture from the fired steam reformer is typically fed to the top of the autothermal reformer and the oxygen-containing gas fed to the burner, mixing and combustion occur downstream of the burner generating a heated gas mixture which is brought to equilibrium as it passes through the steam reforming catalyst. Whereas some steam may be added to the oxygen containing gas, preferably no steam is added so that the low overall steam ratio for the reforming process is achieved. The autothermal reforming catalyst is usually nickel supported on a refractory support such as rings or pellets of calcium aluminate cement, alumina, titanium dioxide, zirconium dioxide and the like. In a preferred embodiment, the secondary reforming catalyst comprises a layer of a higher activity Ni and/or Rh on zirconium dioxide catalyst over a conventional Ni on alumina catalyst to reduce catalyst support volatilisation.

The oxygen-containing gas used in the autothermal reformer of the hydrocarbon reforming unit preferably comprises ≥95% vol. O₂, which may be provided by an air separation unit (ASU) or from another oxygen source. Preferably the O₂ content is ≥98% vol or ≥99% vol. The amount of oxygen-containing gas required in the autothermal reformer is determined by the desired composition of the product gas. In general, increasing the amount of oxygen, thereby increasing the temperature of the reformed gas leaving the autothermal reformer, causes the [H₂] / [CO] ratio to decrease and the proportion of carbon dioxide to decrease. Preferably, the amount of oxygen added is such that the autothermally reformed gas leaves the autothermal reforming catalyst at a temperature in the range 750-1050°C. The autothermally-reformed gas recovered from the autothermal reformer is a synthesis gas comprising hydrogen, carbon monoxide, carbon dioxide, methane and steam. The amount of methane is influenced by the autothermal reformer exit temperature.

The hydrocarbon reforming unit produces a synthesis gas containing hydrogen, carbon monoxide, carbon dioxide and steam. The ideal stoichiometric mixture for methanol synthesis arises when there is enough hydrogen to convert all of the carbon oxides into methanol. The methanol synthesis reactions are as follows:

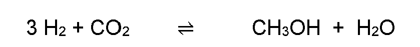

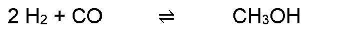

The stoichiometry number or R-value of a synthesis gas may be calculated from the molar composition of the components as follows: R = ([H₂] - [CO₂]) / ([CO] + [CO₂]). The ideal R-value for the methanol synthesis reaction is when R = 2. Accordingly, the synthesis gas recovered from the reforming unit preferably has an R-value in the range 1.95 to 2.05.

After leaving the hydrocarbon reforming unit the synthesis gas is then desirably cooled in one or more steps of heat exchange, generally including at least a first stage of steam raising. Preferably, following such steam raising the reformed gas is cooled by heat exchange with one or more of the following streams; the hydrocarbon feedstock, water (including process condensate), used to generate steam, which may be used for heating or used in the pre-reforming stage, the mixture hydrocarbon and steam, the pre-reformed gas mixture, and in the distillation of crude methanol For safety reasons the reformed gas is preferably not used to heat the oxygen-containing gas fed to the autothermal reformer.

The cooling is preferably performed to lower the temperature of the synthesis gas from the autothermal reformer to below the dew point such that steam present in the synthesis gas condenses. The liquid process condensate may be separated from the synthesis gas, which may be termed make-up gas at this point, by conventional gas-liquid separation equipment.

The make-up gas comprises hydrogen, carbon monoxide, carbon dioxide, and small amount of unreacted methane. The make-up gas may be compressed in a synthesis gas compressor to the desired methanol synthesis pressure for feeding to the methanol loop .

The methanol loop produces an unreacted gas mixture, which is combined with the make-up gas to form a feed gas mixture. In addition, if a hydrogen-rich gas is recovered from the purge gas upstream on the purge gas treatment unit, this hydrogen-rich gas may also be combined with the make-up gas and the unreacted gas mixture to form the feed gas to the methanol loop. The R-value of the feed gas may be in the range 3 to 5 or higher. The addition of a hydrogen-rich gas that has been recovered from the loop purge gas means that the methanol synthesis reactors can be operated at their optimum R value of 3 to 5 with a make-up gas with an R value close to the stoichiometric optimum value of 2 that will maximise the methanol production.

Any methanol loop may be used to synthesise methanol in the process of the present invention. The methanol loop comprises one or more methanol synthesis reactors, for example first, second and optionally third methanol synthesis reactors, each containing a bed of methanol synthesis catalyst, arranged in series and/or parallel that each produce product gas streams containing methanol. The methanol loop may therefore comprise one, two or more methanol synthesis reactors each containing a bed of methanol synthesis catalyst, and each fed with a feed gas comprising hydrogen and carbon dioxide, each producing a gas mixture containing methanol. A product gas mixture containing methanol is recovered from at least one methanol synthesis reactor. Methanol is recovered from one or more of the product gas mixtures. This may be achieved by cooling one or more of the methanol product gas streams to below the dew point, condensing methanol, and separating a crude liquid methanol product from the unreacted gases.

Conventional heat exchange and gas-liquid separation equipment may be used. A particularly suitable heat exchange apparatus includes a gas-gas interchanger that uses a feed gas mixture for a methanol synthesis reactor to cool a methanol product gas stream from that reactor. The methanol product gas streams may be treated separately or may be combined before cooling and/or separating the crude liquid methanol product.

Separation of the crude liquid methanol product from one or more of the methanol product gas streams produces an unreacted gas mixture. A portion of the unreacted gas mixture is returned as a recycle or loop gas stream to one or more of the methanol synthesis reactors. Unreacted gas separated from a product gas mixture recovered from one methanol synthesis reactor may be returned to the same or a different methanol synthesis reactor. The unreacted gas mixture comprises hydrogen, carbon monoxide, and carbon dioxide and so may be used to generate additional methanol. The recycle gas stream may be recovered from at least one of one of the methanol product gas streams and recycled to at least one of the methanol synthesis reactors. If there is more than one recycle gas stream, these may be recycled separately to one or more of the methanol synthesis reactors or combined and fed to one or more of the methanol synthesis reactors.

The methanol synthesis reactor in the methanol loop may be an un-cooled adiabatic reactor. Alternatively, the methanol synthesis reactor may be cooled by heat exchange with a synthesis gas, such as in a quench reactor, or a reactor selected from a tube-cooled converter or a gas-cooled converter. Alternatively, the methanol synthesis reactor may be cooled by boiling water under pressure, such as in an axial-flow steam-raising converter, or a radial-flow steam-raising converter.

In a process comprising first and second methanol synthesis reactors, the first methanol synthesis reactor is preferably cooled by boiling water, such as in an axial-flow steam-raising converter or a radial-flow steam-raising converter, more preferably an axial-flow steam raising converter. The second methanol synthesis reactor may be a radial-flow steam-raising converter. Such arrangements are particularly useful due to the characteristics and performance of these reactors with different feed gas mixtures. Alternatively, the second methanol may be cooled by a synthesis gas, e.g. a gas comprising hydrogen and carbon dioxide. Accordingly, the second methanol synthesis reactor may be a cooled reactor selected from a tube cooled converter (TCC) and a gas-cooled converter (GCC). A tube-cooled converter is preferred because of its simpler design. If a third methanol synthesis reactor is present, it is preferably cooled by boiling water. The third methanol synthesis reactor may then suitably be a steam-raising converter selected from an axial-flow steam-raising converter and a radial-flow steam-raising converter, most preferably an axial-flow steam raising converter. The first and second methanol synthesis reactors may be connected in series in which case the synthesis gas fed to the second methanol synthesis reactor comprises at least a portion of a methanol product gas stream recovered from the first methanol synthesis reactor. In such an arrangement, preferably the synthesis gas fed to the second methanol synthesis reactor comprises all of the methanol product gas stream recovered from the first methanol synthesis reactor. Particularly preferred methanol loops are described in US7790775, WO2017/121980 A1 and WO2017/121981 A1.

The methanol synthesis catalysts in each of the methanol synthesis reactors may be the same or different. The methanol synthesis catalysts are preferably copper-containing methanol synthesis catalysts, which are commercially available. In particular, the methanol synthesis catalysts are one or more particulate copper/zinc oxide/alumina catalysts, which may comprise one or more promoters. Particularly suitable catalysts are Mg-promoted copper/zinc oxide/alumina catalysts as described in US4788175 and SiO₂-doped copper/zinc oxide/alumina catalysts as described in WO2020/212681 A1.

Methanol synthesis may be effected in the one or more methanol synthesis reactors at pressures in the range 10 to 120 bar abs, and temperatures in the range 130°C to 350°C. The pressures at the one or more reactor inlets is preferably 50-100 bar abs, more preferably 70-90 bar abs. The temperature of the synthesis gas at the one or more reactor inlets is preferably in the range 200-250°C and at the one or more reactor outlets preferably in the range 230-280°C.

The portion of the unreacted gas mixture making up the recycle gas stream to the methanol loop will typically be at a lower pressure than the make-up gas and so preferably the recycle gas stream is compressed by one or more compressors or circulators. At least one compressor is used to circulate the unreacted gas stream. The resulting compressed recycle gas stream may be mixed with make-up gas to form the feed to the one or more methanol synthesis reactors in the methanol loop.

The recycle ratios to form the feed gas mixtures to the one or more methanol synthesis reactors may be in the range 0.5:1 to 5:1 preferably 1:1 to 3:1. By the term "recycle ratio", we mean the molar flow ratio of the recycled unreacted gas stream to the make-up gas that form the gas mixtures fed to the one or more methanol synthesis reactors.

The crude liquid methanol recovered from the methanol loop contains water, along with small amounts of higher alcohols and other impurities. The crude methanol may first be fed to a flash column or let-down vessel, where dissolved gases are released and separated from the crude liquid methanol stream. The crude liquid methanol may also be subjected to one or more purification stages including one or more, preferably two or three, stages of distillation in a methanol purification unit comprising one, two or more distillation columns. Off-gases may be usefully recovered from the let-down vessel and at least the topping column in the distillation unit and fed to the purge gas treatment unit, to further reduce carbon dioxide emissions from the process. The de-gassing stage and distillation stages may be heated using heat recovered from the process, for example in the cooling of a product gas stream, or by other sources. Preferably at least a portion of the crude methanol is purified by distillation to produce a purified methanol product.

The purified methanol product may be subjected to further processing, for example to produce derivatives such as dimethyl ether or formaldehyde. Alternatively, the methanol may be used as a fuel.

A portion of the unreacted gas mixture separated from the crude liquid methanol is removed from the loop as the purge gas stream. The purge gas stream is preferably removed continuously to prevent the unwanted build-up of inert gases, such as nitrogen, argon and methane in the synthesis loop. The purge gas stream may be recovered from the separated unreacted gases before or after compression in the circulator. Purge gas streams in processes using steam reforming as a source of the make-up gas may be hydrogen rich. The purge gas stream preferably contains 50-90% by volume of hydrogen and one or more of carbon monoxide, carbon dioxide, nitrogen, argon and methane.

In the present invention, at least a portion of the purge gas stream is subjected to partial oxidation or reforming in a purge gas reforming unit to form a partially-oxidised or reformed purge gas followed by one or more stages of water gas shift of the partially-oxidised or reformed purge gas in a water-gas shift unit to form a hydrogen-enriched gas and a step of carbon dioxide removal from the hydrogen-enriched gas in a carbon dioxide removal unit to form a hydrogen stream and a carbon dioxide stream, the carbon dioxide stream is recovered and at least a portion of the hydrogen stream is fed to the fired steam reformer as a fuel.

The invention may therefore include installation into an existing methanol production unit comprising a fired steam reformer of a purge gas treatment unit comprising a partial oxidation reactor or a purge gas reforming unit configured to provide a partially-oxidised or reformed purge gas, a water gas shift unit comprising one or more water-gas shift reaction vessels configured to provide a hydrogen-enriched gas, a carbon dioxide removal unit configured to provide a hydrogen stream and a carbon dioxide stream, and means to convey at least a portion of the hydrogen stream to the fired steam reformer as a fuel.

If the hydrocarbon reforming unit consists of a fired steam reformer it is possible, if pure oxygen is employed in the purge gas treatment unit in the partial oxidation unit or autothermal reformer, to recover a portion of the partially oxidised or reformed gas upstream of the water-gas shift unit, optionally cool it, and add it to an existing and/or new methanol loop. This provides the possibility of building a new oxygen based autothermal reformer methanol production unit in parallel to an existing fired reformer-based methanol production facility, which not only uses the advantage of the existing methanol purge stream to add cheaper methanol production capacity, but also decarbonises an existing production facility.

The purge gas stream may contain methanol and so, if desired, upstream of the purge gas treatment unit, methanol may optionally be recovered from the purge gas stream using a water wash, and the recovered methanol and water sent for purification with the crude methanol.

At least a portion of the purge gas may be fed directly to the purge gas treatment unit, for example where the synthesis gas generated by the hydrocarbon reforming unit has an R-value of 1.95 or higher. This may be the case, for example, where the reformer in the hydrocarbon reforming unit consists of a fired steam reformer.

Alternatively, a hydrogen-rich gas stream may be separated from the purge gas upstream of the purge gas treatment unit thereby forming a carbon-rich purge gas which is fed to the purge gas treatment unit. This may be the case, for example, where the synthesis gas from the hydrocarbon reforming unit has an R-value of less than 1.95. Then is advantageous to recover hydrogen from at least a portion of the purge gas and feed that hydrogen to the methanol loop. This may be preferred where the hydrocarbon reforming unit comprises both a fired steam reformer and an autothermal reformer, and a portion of the hydrocarbon feedstock is fed to the autothermal reformer. In these arrangements, the purge gas may be separated into a hydrogen-rich gas and a carbon-rich purge gas. By "carbon-rich purge gas" we mean a gas stream that has a higher proportion of carbon-containing compounds (carbon monoxide, carbon dioxide and methane) than the parent purge gas. While individual components may have the same, or even lower, proportion than in the parent purge gas, the total of all carbon-containing compounds will be higher in the carbon-rich purge gas. Consequently, in such arrangements the portion of the purge gas fed to the purge gas treatment unit is the carbon-rich purge gas. Separating hydrogen from the purge gas upstream of the purge gas treatment unit also has the advantage that it reduces the loss of hydrogen from the process by combustion in the purge gas treatment unit. The separation of the hydrogen-rich and carbon-rich purge gas streams may be practiced using known separation equipment such as hydrogen membrane separator or a pressure swing adsorption unit, a cold box separation system or any combination of these. Using these techniques over 50% of the hydrogen present in the parent purge gas stream may be recovered. Where a membrane is used to separate the hydrogen-rich gas, the carbon-rich purge gas stream will be at a pressure that enables it to be sent for partial oxidation or reforming in the purge gas treatment unit without further compression. This is highly desirable. Where a pressure swing absorption system is used to separate the hydrogen-rich gas, the carbon-rich purge gas may be at a lower pressure and so is less preferred. The hydrogen-rich gas stream recovered from the purge gas stream desirably comprises >95% by volume of H₂. The separated hydrogen, in addition to being fed to the methanol loop may also be used upstream in hydrodesulphurisation of the hydrocarbon feedstock and/or used to strip dissolved gases from the crude methanol, and/or be used as fuel. However, preferably at least 90% by volume of the separated hydrogen-rich gas stream is fed to the methanol loop.

The feed to the purge gas treatment unit may be supplemented with a portion of the hydrocarbon feedstock. This increases the flexibility of the purge gas treatment unit, ensures there is sufficient hydrogen for firing the fired steam reformer, and is advantageous during start-up of the process. If desired, upstream of the purge gas treatment unit, the portion of the hydrocarbon feedstock used to supplement the feed may be pre-reformed using an adiabatic pre-reformer as described above.

The feed to the purge gas treatment unit may, if desired, be supplemented with another off-gas from the process, for example a let-down vessel off-gas and/or a distillation off-gas.

The portion of the purge gas is fed to the partial oxidation reactor or the purge gas reforming unit in the purge gas treatment unit. This may advantageously be 100% by volume of the purge gas or the carbon-rich purge gas.

The purge gas reforming unit in the purge gas treatment unit may comprise an autothermal reformer. The scale of the autothermal reformer in the purge gas reforming unit may be the same or different to that in the hydrocarbon reforming unit, but the design, catalyst and operation are conveniently the same as described above. The portion of the purge gas fed to the autothermal reformer may be compressed, if necessary, to a pressure in the range of 10-50 bar abs and heated, if necessary, to a temperature of 350-650°C prior to being fed to the autothermal reformer. Heating of the portion of the purge gas may be performed using a fired heater that uses a portion of the hydrogen stream produced by the purge gas treatment unit as fuel. The oxygen-containing gas fed to the partial oxidation reactor or autothermal reformer in the purge gas reforming unit may be air, oxygen enriched air or oxygen gas as an oxidant, but is preferably air. The oxygen-containing gas may be the same or different from that used in any autothermal reformer in the hydrocarbon reforming unit, although it is convenient to use air because the resulting hydrogen stream is used as fuel and the presence of nitrogen may be tolerated. The use of air avoids the need to uprate or add a further air separation unit where an existing process is being retrofitted with a purge gas treatment unit. Steam may be added to the oxygen-containing gas and/or the purge gas portion. The purge gas is autothermally reformed in the autothermal reformer to produce a reformed purge gas. The reformed purge gas will comprise hydrogen, steam carbon monoxide, carbon dioxide and possibly small amounts of argon and nitrogen.

A partial oxidation reactor may alternatively be used to convert the portion of the purge gas into a partially oxidised purge gas by partial oxidation using a sub-stoichiometric amount of oxygen. Partial oxidation reactors, or POx reactors, are known and typically comprise a vessel to which the feed and an oxygen-containing gas are fed via a burner, analogous to that used in an autothermal reformer, disposed above a reaction chamber in which the partial combustion reactions take place. Unlike an autothermal reformer, a catalyst is not present in the vessel. The oxygen-containing gas may be the same or different from that used in any autothermal reformer in the hydrocarbon reforming unit, although it is convenient to use air for the reasons set out above for the autothermal reformer. The combustion temperature may be about 1300°C, or higher. Steam may be added to the feed and/or the oxygen lower the combustion temperature and reduce soot formation. The idealised formula for this reaction applied to methane in the feed is as follows:

CH₄ + ½ O₂ → CO + 2 H₂

However, yields are below stoichiometric because part of the feed is fully combusted, and the problem of soot formation requires generally increased amounts of steam or oxygen, and so the purge reforming unit preferably comprises an autothermal reformer rather than a partial oxidation reactor.

The exit temperature from a purge gas autothermal reformer or purge gas partial oxidation reactor may be in the range 800-1300°C. It is desirable therefore to adjust the temperature of the partially-oxidised or reformed purge gas upstream of the water gas shift unit. This may conveniently be done by recovering heat in a heat recovery unit, including the generation of steam in one or more boilers, which steam may usefully be used in heating or in power generation using a steam turbine. In some embodiments, steam generated by the heat recovery from the purge gas reforming unit may be used to supplement the steam addition to the hydrocarbon feedstock and/or purge gas upstream of the respective reforming units. Extra steam generated in the purge gas treatment unit may be used to provide process heating or motive power for compressors or for generating electricity.

The partially-oxidised or reformed purge gas is subjected to one or more stages of water-gas shift in a water- gas shift unit. Steam is necessary for the water-gas shift reaction. If insufficient steam is present in the partially-oxidised or reformed purge gas, steam may be added upstream of the water gas shift unit, e.g. by direct addition.

The partially-oxidised or reformed purge gas may be passed through one or more beds of water-gas shift catalyst in one or more shift vessels to generate a hydrogen-enriched, or "shifted", gas. At the same time the water gas shift unit converts carbon monoxide in the partially-oxidised or reformed purge gas to carbon dioxide. The reaction may be depicted as follows;

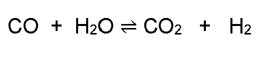

The one or more water-gas shift stages may include stages of high-temperature shift, medium-temperature shift, isothermal shift and low-temperature shift.

High-temperature shift may be operated adiabatically in a shift vessel at inlet temperatures in the range 300-400°C, preferably 320-360°C, over a bed of a reduced iron catalyst, such as chromia-promoted magnetite. Alternatively, a potassium promoted zinc-aluminate catalyst may be used. A single stage of high-temperature shift may be used in the present invention. Alternatively, a combination of high-temperature and medium- temperature or low-temperature shift may be used

Medium-temperature shift and low-temperature shift stages may be performed using shift vessels containing supported copper-catalysts, particularly copper/zinc oxide/alumina compositions. In low-temperature shift, a gas containing carbon monoxide (preferably ≤ 6% vol CO on a dry basis) and steam (at a steam to total dry gas molar ratio in range 0.3 to 1.5) may be passed over the catalyst in an adiabatic fixed bed with an outlet temperature in the range 200 to 300°C. The outlet carbon monoxide content may be in the range 0.1 to 1.5%, especially under 0.5% vol on a dry basis if additional steam is added. Alternatively, in medium-temperature shift, the gas containing carbon monoxide and steam may be fed to the catalyst at an inlet temperature in the range 200 to 240°C although the inlet temperature may be as high as 280°C. The outlet temperature may be up to 300°C but may be as high as 360°C.

Whereas one or more adiabatic water-gas shift stages may be employed, such as a high-temperature shift stage, optionally followed by a low-temperature shift stage, the partially-oxidised or reformed purge gas may be subjected to a stage of isothermal water-gas shift in a shift vessel in which the catalyst is cooled, optionally followed by one or more adiabatic medium- or low-temperature water-gas shift stages in un-cooled vessels as described above. Whereas the term "isothermal" is used to describe a cooled shift converter, there may be a small increase in temperature of the gas between inlet and outlet, so that the temperature of the hydrogen-enriched reformed gas stream at the exit of the isothermal shift converter may be between 1 and 25 degrees Celsius higher than the inlet temperature. The coolant conveniently may be water under pressure such that partial, or complete, boiling takes place. The water can be in tubes surrounded by catalyst or vice versa. The resulting steam can be used in the process, for example, to drive a turbine, e.g. for electrical power, or to provide process steam for supply to the process. In some embodiments, steam generated by the isothermal shift stage may be used to supplement the steam addition to the hydrocarbon feedstock upstream of the hydrocarbon reforming unit and/or purge gas upstream of the purge gas treatment unit. This improves the efficiency of the process and enables the desired steam to carbon ratio to be achieved at low cost.

Following the one or more shift stages, the hydrogen-enriched reformed gas is desirably cooled to a temperature below the dew point so that the steam condenses. This forms a de-watered hydrogen-enriched gas. The liquid water condensate may then be separated using one or more, gas-liquid separators, which may have one or more further cooling stages between them. Any coolant may be used. Typically cooling of the hydrogen-enriched gas may be provided by boiling water under pressure coupled to a steam drum. If desired, cooling may be carried out in heat exchange with the process condensate. As a result, a stream of heated water, which may be used to supply some or all of the steam required for partial oxidation or reforming in the hydrocarbon reforming unit and/or the purge gas reforming unit, may be formed. Because the condensate may contain ammonia, methanol, hydrogen cyanide and CO₂, returning the condensate to form steam used in the partial oxidation or reforming stage offers a useful way of returning hydrogen and carbon to the process. One or more further stages of cooling are desirable. The cooling may be performed in heat exchange in one or more stages using demineralised water, air, or a combination of these. In a preferred embodiment, cooling is performed in heat exchange with one or more liquids used in the CO₂ separation unit. One, two or three stages of condensate separation may be performed. Any condensate not used to generate steam may be sent to water treatment as effluent.

Typically, the hydrogen-enriched gas stream contains 10 to 30% vol of carbon dioxide (on a dry basis). In the present invention, preferably after separation of the condensed water, carbon dioxide is separated from the hydrogen-enriched gas stream in a carbon dioxide removal unit.

The carbon dioxide removal unit may operate by means of adsorption of carbon dioxide into a solid adsorbent, such as a molecular sieve, in a pressure swing absorption (PSA) unit, separation of a hydrogen-rich gas using a hydrogen -permeable membrane, or alternatively by absorption into a liquid in a physical wash system or a reactive wash system. Solid adsorbent and membrane systems may be used where the amount of purge gas and/or the purity of the hydrogen stream are not high. However, for improved carbon dioxide removal, a reactive wash system, especially an amine wash system, is preferred. The carbon dioxide may therefore be separated by an acid gas recovery (AGR) process. In the AGR process, a de-watered hydrogen-enriched reformed gas stream (i.e. a de-watered shifted gas) is contacted with a stream of a suitable absorbent liquid, such as an amine, for example monoethanolamine, diethanolamine, methyl diethanolamine and diglycolamine, particularly methyl diethanolamine (MDEA) solution so that the carbon dioxide is absorbed by the liquid to give a laden absorbent liquid and a gas stream having a decreased content of carbon dioxide. The laden absorbent liquid is then regenerated by heating, to desorb the carbon dioxide and to give a regenerated absorbent liquid, which is then recycled to the carbon dioxide absorption stage. The heating may suitably be provided by steam, hot condensate or another suitable heating medium generated by the process. Alternatively, chilled methanol or a glycol may be used to capture the carbon dioxide in a similar manner as the amine. If the carbon dioxide separation step is operated using a liquid washing step as a single pressure process, i.e. essentially the same pressure is employed in the absorption and regeneration steps, only a little recompression of the recycled carbon dioxide will be required. Carbon dioxide removal units of the types described above are commercially available.

Because the source of the hydrogen-enriched gas is a purge gas stream, inert substances such as nitrogen and argon may be present. An amine wash carbon dioxide removal unit conveniently leaves these inert gases within the hydrogen gas stream that is fed to the fired steam reformer as fuel, so in this way they may be effectively removed from the process.

The recovered carbon dioxide is relatively pure and so may be compressed and used for the manufacture of chemicals, purified for use in the food industry, or sent to storage or sequestration or used in enhanced oil recovery (EOR) processes. In cases where the CO₂ is to be compressed for storage, transportation, use in EOR processes or conversion to other chemical products, the CO₂ may be first dried to prevent liquid water present in trace amounts, from condensing. For example, the CO₂ may be dried to a dew point ≤ 10°C by passing it through a bed of a suitable desiccant, such as a zeolite, or contacting it with a glycol in a glycol drying unit.

Upon the separation of the carbon dioxide, the process provides a hydrogen gas stream. Where a pure oxygen-containing gas stream (i.e. ≥95% vol, preferably ≥98% O₂ vol) is used in the partial oxidation reactor or autothermal reformer in the purge gas reforming unit, the hydrogen stream may comprise 75-99% vol hydrogen, preferably 90-99% vol hydrogen, with the balance comprising one or more of methane, carbon monoxide, carbon dioxide and inert gases. Where_air is used in the partial oxidation reactor or autothermal reformer in the purge gas reforming unit, the hydrogen stream may comprise 50-60% vol hydrogen, with the balance comprising mostly nitrogen with minor amounts of one or more of methane, carbon monoxide, carbon dioxide and argon. The methane content of the hydrogen stream may be in the range 0.25-7.5% vol. The carbon monoxide content of the hydrogen stream may be in the range 0.5-7.5% vol. The carbon dioxide content of the hydrogen stream may be in the range 0.01-2.5% vol.

When the purity of the hydrogen stream required for downstream purposes needs to be higher than produced by the carbon dioxide removal unit, the hydrogen gas stream may be passed to a purification unit to provide a purified hydrogen product stream and an off-gas stream containing carbon compounds.

The purification unit may comprise a membrane system, a temperature swing adsorption system, or a pressure swing adsorption system. The purification unit is preferably a pressure-swing adsorption unit. Such units comprise regenerable porous adsorbent materials that selectively trap gases other than hydrogen and thereby purify it. The purification unit produces a pure hydrogen stream preferably with a purity greater than 99.5% vol, more preferably greater than 99.9% vol. Such systems are commercially available. The purification unit also produces an off gas. The off gas contains carbon compounds and so is preferably not used as a fuel, but rather is fed back into the process as a feed, either to the hydrocarbon reforming unit or, preferably, the purge gas treatment unit.

Carbon dioxide recovered from the carbon dioxide removal unit may be optionally purified, compressed, and sent for storage or sequestration. Alternatively, at least a portion of the carbon dioxide may be sent to other processes that utilise carbon dioxide as a feed. If desired, depending on the stoichiometry of the feed to the methanol loop, it may be desirable to feed a portion of the recovered carbon dioxide to the methanol loop to generate additional methanol. For example, where the feed gas to the methanol loop has a stoichiometry figure R above 2.05, e.g. in the range 2.1 to 3.0, it may be desirable to recover a portion of the carbon dioxide from the carbon dioxide removal unit and feed it to the synthesis gas upstream of the one or methanol synthesis reactors of the methanol loop to adjust the stoichiometry towards the desired 1.95 to 2.05 range. The adjustment of the stoichiometry of the methanol synthesis gas using recovered carbon dioxide increases methanol production but reduces the amount of purge gas going to the purge gas treatment unit, however this could be accommodated by adding extra hydrocarbon feed to the purge gas treatment unit. If recovered carbon dioxide is added to the methanol synthesis gas, this may enable the hydrocarbon reforming unit to operate at a lower outlet temperature, which increases throughput. Whereas a lower outlet temperature increases the methane content of the synthesis gas and in consequence can make the methanol loop less efficient, because additional methanol is made in the methanol loop, this can be accommodated. The present invention therefore potentially provides a revamp strategy with two aspects; to decarbonise the existing production unit and to increase methanol production from the existing production unit.

If desired, a portion of the crude hydrogen or a portion of the purified hydrogen may be compressed, if necessary, and recycled to the hydrocarbon feed for hydrodesulphurisation and to reduce the potential for carbon formation on the catalyst in the fired steam reformer.

The fired steam reformer is fired using at least a portion of the hydrogen product produced by the purge gas treatment unit. This offers a potential reduction in CO₂ emissions from an existing process using purge gas and natural gas mixtures as fuel of at least 90% and potentially 95%, or higher. The replacement of the conventional carbon-containing fuel gas may require adjustment of one or more of the burners in the fired steam reformer, or replacement of one or more of the burners. Therefore, where an existing fired reformer is retrofitted with a purge gas treatment unit as described above, the retrofitting method may include installation of new H₂ fuel burners in the fired reformer. In addition, adjustment may be needed in a convection section or heat recovery duct of the fired steam reformer. This may include adding extra burners in the heat recovery duct to satisfy fired reformer heat demand. If one or more fired heaters are installed, these are desirably fired by a portion of the hydrogen product to maintain the advantage of the purge gas treatment unit in decarbonising the methanol process. Furthermore, where the use of the hydrogen stream as fuel in the fired steam reformer results is less heat recovery in the convection section or heat recovery duct of the fired reformer, then a fired heater in the purge gas treatment unit using a portion of the hydrogen stream as fuel advantageously may be used to make up the balance. Accordingly, the invention includes providing the purge gas treatment unit with a fired heater to ensure the heating demand of overall plant is satisfied.

In order to minimise emissions of nitrogen oxides from the fired reformer and any fired heater used in the purge gas treatment unit, it is desirable that either, or both, have flue-gas purification unit installed that removes or decomposes the nitrogen oxides that may be formed by the combustion of the hydrogen stream with air. Such "de-NOx" purification units are known and are commercially available.

The purge gas treatment unit may be fed by more than one methanol synthesis unit and may also returns the hydrogen stream to more than one hydrocarbon reforming unit. The purge gas treatment unit may therefore be operatively connected to two or more methanol production units each having a hydrocarbon reforming unit containing a fired reformer. Furthermore, in the present invention, it is not necessary that the purge gas treatment unit is located adjacent the methanol production unit.

The invention will now be further illustrated by reference to the Figures in which;
Figure 1 is a flow sheet depicting a methanol production unit according to one embodiment of the invention comprising a fired steam reformer and a purge gas treatment unit, with hydrogen product supplied as fuel for the fired steam reformer, and
Figure 2 is a flow sheet depicting one embodiment of a purge gas treatment unit suitable for use in the present invention.

It will be understood by those skilled in the art that the drawings are diagrammatic and that further items of equipment such as feedstock drums, pumps, vacuum pumps, compressors, gas recycling compressors, temperature sensors, pressure sensors, pressure relief valves, control valves, flow controllers, level controllers, holding tanks, storage tanks and the like may be required in a commercial plant. Provision of such ancillary equipment forms no part of the present invention and is in accordance with conventional chemical engineering practice.

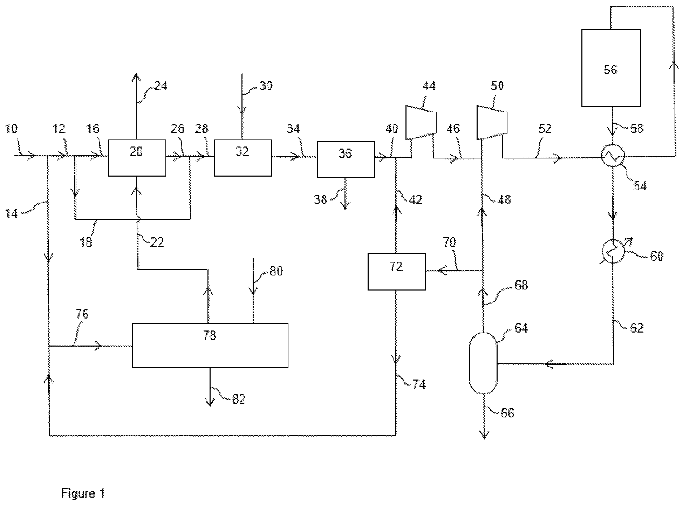

In Figure 1, a natural gas feed supplied by line 10 is divided into a first portion 12 and a second portion 14. The first portion is divided into a fired steam reformer feed stream 16 and a fired steam reformer bypass stream 18. The fired steam reformer feed stream 16 is combined with steam using a saturator or by direct steam addition (not shown), optionally subjected to a step of adiabatic pre-reforming (not shown) and fed to a hydrocarbon reforming unit comprising a fired steam reformer 20 containing a plurality of externally heated catalyst-filled reformer tubes. The fired steam reformer is heated by combustion of a fuel supplied by line 22 and generates a flue gas emitted via line 24. Steam reforming reactions take place as the natural gas and steam mixture passes over the steam reforming catalyst in the reformer tubes to generate a crude synthesis gas comprising hydrogen, carbon monoxide, carbon dioxide, unreacted methane and steam. The synthesis gas is recovered from the fired steam reformer 20 via line 26 and combined with the fired steam reformer bypass stream 18 to form a secondary reformer feed gas mixture 28. The secondary reformer feed gas mixture 28 and an oxygen gas stream provided by line 30 from an air separation unit (not shown) are fed to the burner of an autothermal reformer 32, where partial combustion takes place generating a hot gas mixture that is adiabatically reformed in a bed of steam reforming catalyst disposed below the burner, to generate a synthesis gas mixture comprising hydrogen, carbon monoxide, carbon dioxide and steam.

The synthesis gas is recovered from the autothermal reformer 32 at a temperature above 900°C via line 34 and passed to a heat recovery unit 36, where the synthesis gas is cooled in two or more stages by heat exchange in an interchanger with a process stream, and/or using water and air as coolant to cool the synthesis gas to below the dew point such that the steam condenses. In the heat recovery unit 36, condensate is recovered from cooled synthesis gas using one or more gas liquid separators (not shown) to generate a condensate stream 38, which is used as a source of steam used in the steam reforming stages of the process.

Separation of the condensate generates a make-up gas, which is recovered from the heat recovery unit 36 via line 40, mixed with a hydrogen-enriched gas stream provided by line 42, and the resulting hydrogen-enriched make-up gas compressed in syngas compressor 44. The compressed hydrogen-enriched make-up gas 46 is then combined with a recycle stream of unreacted gas provided by line 48 and the combined feed gas fed to a circulating loop compressor 50. The compressed feed gas is pre-heated in interchanger 54 and fed to a methanol synthesis unit 56 comprising one or more methanol synthesis reactors containing a methanol synthesis catalyst. The methanol synthesis unit 56 may comprise one, two or more methanol synthesis reactors, which may be cooled or uncooled, and connected in parallel or series. Methanol synthesis reactions take place over the methanol synthesis catalyst to convert hydrogen, carbon monoxide and carbon dioxide to a gaseous methanol product mixture comprising methanol and steam.

The gaseous methanol product mixture is recovered from the methanol synthesis unit 56 via line 58, cooled in interchanger 54 and then in one or more further stages of cooling in heat exchangers 60 to below the dew point at which the methanol and steam condense. The cooled mixture is then fed via line 62 to a gas-liquid separator 64 that separates a liquid crude methanol stream from the unreacted gas. Crude methanol is recovered from the separator 64 via line 66 and sent for purification to provide a purified methanol product. The unreacted gas is recovered from the separator 64 via line 68. A purge gas stream is taken from line 68 via line 70 and the remaining unreacted gas fed to the hydrogen-enriched make-up gas via line 48.

The purge gas stream 70 is fed to a hydrogen separation unit 72 in which the purge gas stream is separated into a hydrogen-rich stream and a carbon-rich purge gas stream by passing the purge gas stream through a suitable membrane. The hydrogen-rich gas stream is recovered from the separation unit 72 via line 42 and mixed with the make-up gas in line 40 to form the hydrogen-enriched make-up gas.

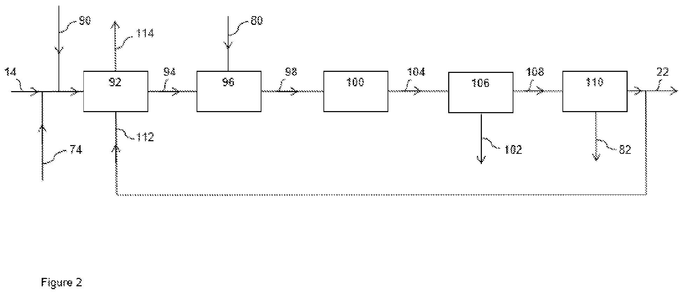

The carbon-rich purge gas stream is recovered from the separation unit 72 by line 74, combined with the second portion 14 of the natural gas, and fed via line 76 to a purge gas treatment unit 78. In the purge gas treatment unit 78, further described by reference to Figure 2, the combined carbon-rich purge gas stream and natural gas mixture are heated and passed to a purge gas reforming unit in which it is subjected to autothermal reforming with an oxygen-containing gas, such as air, oxygen-enriched air or oxygen gas fed via line 80 in a purge gas autothermal reformer, then heat recovery and optional condensate separation in a heat recovery unit, followed by water-gas shift in a water gas shift unit, and finally CO₂ removal in a carbon dioxide removal unit. The CO₂ removal unit generates a carbon dioxide stream, which is recovered from the purge gas treatment unit 78 by line 82, optionally purified, compressed, and sent for storage or sequestration. The removal of CO₂ generates a hydrogen stream, which is recovered from the purge gas treatment unit 78 and fed via line 22 as fuel to the fired steam reformer 20, thereby generating a low CO₂ flue gas 24.

In Figure 2, one embodiment of a suitable purge gas treatment unit is depicted. The second portion 14 of the natural gas feed, optionally after a step of adiabatic pre-reforming, is combined with the carbon rich gas 74. Other carbon-containing gases, such as an off gas from a let-down vessel and/or distillation overheads may be compressed and combined with the feed gas. Steam is optionally added from line 90 and the resulting mixture heated in a fired heater 92. The fired heater 92 is heated by combustion of a hydrogen stream 112 with air to produce a combustion flue gas 114.

The heated gas mixture is fed via line 94 to a purge gas reforming unit comprising a purge gas autothermal reformer 96, where it is partially combusted in a burner with the oxygen-containing gas fed via line 80. The oxygen containing gas fed to the purge gas autothermal reformer 96 may be air and/or a portion of the oxygen containing gas recovered from the air separation unit used to provide the oxygen containing gas stream 30. The partially combusted gas is then adiabatically steam reformed in a bed of steam reforming catalyst disposed beneath the burner within the autothermal reformer 96. The autothermal reforming generates a reformed purge gas comprising hydrogen, carbon monoxide, carbon dioxide and steam, which is fed via line 98 to a heat recovery unit (not shown) to reduce the temperature. In one arrangement, the cooling reduces the temperature of the reformed purge gas to between 200 and 300°C and above the dew point, such that the cooled reformed gas may be fed directly, after optional steam addition, to a water-gas shift unit 100. Less preferably, the reformed purge gas may be cooled to below the dew point such that the steam condenses, and condensate recovered using one or more gas liquid separators and used as a source of steam for the process. Then, after heating and steam addition the de-watered reformed gas may be fed to the water gas shift unit 100.

The cooled reformed purge gas from the heat recovery unit is passed to the water gas shift unit 100, desirably comprising an isothermal shift vessel containing an isothermal shift catalyst in which the reformed purge gas becomes enriched in hydrogen by the water-gas shift reaction to form a hydrogen-enriched gas stream.

The hydrogen-enriched reformed gas recovered from the water gas shift unit 100 is then fed via line 104 to a heat recovery unit 106 that cools the hydrogen-enriched gas to below the dew point such that remaining steam condenses. The heat recovery unit 106 comprises one of more gas liquid separators that separate the condensate, which is recovered via line 102 for use in the process.

The resulting dewatered hydrogen-enriched gas is fed from the heat recovery unit 106 via line 108 to a carbon dioxide removal unit 110 operating my means of an amine wash, which absorbs carbon dioxide from the dewatered hydrogen-enriched gas to produce a hydrogen stream. The hydrogen stream is recovered from the carbon dioxide removal unit 110 and divided between the portion 112 fed to the fired heater 92 and the portion 22 fed to the fired steam reformer 20. Regeneration of the amine absorbent in the carbon dioxide removal unit 110 generates a carbon dioxide stream, which is recovered from the unit 110 via line 82. The recovered carbon dioxide may be compressed and sent for sequestration.

In a retrofit of an existing plant with hydrogen recovery, instead of feeding the carbon rich off gas to the fired steam reformer as a fuel, it is treated in an installed purge gas treatment unit to generate a carbon dioxide stream which is recovered, and a hydrogen stream which is used as fuel in the fired steam reformer and any fired heaters.

The invention will be further described by reference to the following calculated examples prepared using conventional process modelling software suitable for methanol processes.

### Example 1

Example 1 is an example of a flowsheet according to Figure 1 using the purge gas treatment unit of Figure 2, designed to produce 5000 tonnes/day methanol. The process conditions and compositions of the various streams are set out below.

| Stream Number | | 14 | 22 | 24 | 42 | 66 | 70 |
|---|---|---|---|---|---|---|---|
| Temperature | °C | 331 | 40 | 150 | 71 | 50 | 55 |
| Pressure | bar a | 52.0 | 14.5 | 1.02 | 39.6 | 76.4 | 75.2 |
| Mass Flow | tonne/h | 10.73 | 8.526 | 237.9 | 13.04 | 241.9 | 26.03 |
| Molar Flow | kgmole/h | 637.0 | 2983 | 9566 | 1512 | 8110 | 2294 |
| Molecular Weight | | 16.84 | 2.86 | 24.87 | 8.63 | 29.83 | 11.35 |
| Composition | kgmole/h | | | | | | |
| Water | | 0.1 | 10.8 | 3234.1 | 6.4 | 1236.3 | 7.2 |
| Hydrogen | | 19.1 | 2884.8 | - | 1120.0 | 20.9 | 1317.5 |
| Carbon Monoxide | | 2.8 | 27.2 | - | 74.9 | 16.9 | 190.1 |
| Carbon Dioxide | | 5.0 | - | 38.6 | 108.5 | 131.9 | 148.1 |
| Nitrogen | | 3.8 | 31.8 | 6055.6 | 16.7 | 3.0 | 48.3 |
| Argon | | 0.4 | 17.0 | 94.2 | 14.9 | 2.6 | 28.9 |
| Methane | | 575.8 | 11.2 | - | 170.7 | 107.5 | 553.6 |
| Ethane | | 23.4 | - | - | - | - | - |
| Propane | | 4.8 | - | - | - | - | - |
| Butane | | 1.4 | - | - | - | - | - |
| Pentane + | | 0.4 | - | - | - | - | - |
| Methanol | | - | - | - | - | 6580.4 | - |
| Oxygen | | - | - | 143.5 | - | - | - |
| Lights | | - | - | - | - | 3.3 | 0.3 |
| Heavies | | - | - | - | - | 7.1 | - |

| Stream Number | | 74 | 80 | 82 | 90 | 94 | 98 |
|---|---|---|---|---|---|---|---|
| Temperature | °C | 71 | 20 | 47 | 240 | 580 | 950 |
| Pressure | bar a | 72.0 | 50.0 | 1.30 | 33.3 | 19.6 | 18.8 |
| Mass Flow | tonne/h | 12.99 | 22.19 | 65.07 | 51.16 | 83.90 | 106.3 |
| Molar Flow | kgmole/h | 782.1 | 692.6 | 1565 | 2840 | 4565 | 6874 |
| Molecular Weight | | 16.61 | 32.04 | 41.57 | 18.02 | 18.38 | 15.47 |
| Composition | kgmole/h | | | | | | |
| Water | | 0.8 | - | 131.4 | 2840.0 | 2842.3 | 2702.1 |
| Hydrogen | | 197.6 | - | 9.4 | - | 237.8 | 2641.0 |
| Carbon Monoxide | | 115.2 | - | 0.2 | - | 134.9 | 872.9 |
| Carbon Dioxide | | 39.6 | - | 1423.6 | - | 176.8 | 585.3 |
| Nitrogen | | 31.6 | - | 0.1 | - | 38.4 | 38.4 |
| Argon | | 14.1 | 3.5 | 0.1 | - | 17.1 | 20.6 |
| Methane | | 382.9 | - | 0.2 | - | 1066.5 | 13.7 |
| Ethane | | - | - | - | - | 23.4 | - |
| Propane | | - | - | - | - | 4.8 | - |
| Butane | | - | - | - | - | 1.4 | - |
| Pentane + | | - | - | - | - | 0.4 | - |
| Methanol | | - | - | - | - | 18.0 | - |
| Oxygen | | - | 689.1 | - | - | - | - |
| Lights | | 0.3 | - | - | - | 3.2 | - |
| Heavies | | - | - | - | - | - | - |

| I Stream Number | | 102 | 104 | 108 | 112 | 114 |
|---|---|---|---|---|---|---|
| Temperature | °C | 65 | 244 | 65 | 40 | 153 |
| Pressure | bar a | 14.7 | 16.0 | 14.7 | 14.5 | 1.02 |
| Mass Flow | tonne/h | 31.95 | 106.3 | 74.39 | 1.736 | 53.09 |
| Molar Flow | kgmole/h | 1771 | 6874 | 5103 | 607.2 | 2095 |
| Molecular Weight | | 18.04 | 15.47 | 14.58 | 2.86 | 25.34 |
| Composition | kgmole/h | | | | | |
| Water | | 1769.6 | 1862.1 | 92.3 | 2.2 | 624.9 |
| Hydrogen | | - | 3481.0 | 3481.1 | 587.2 | - |
| Carbon Monoxide | | - | 32.9 | 32.9 | 5.5 | - |
| Carbon Dioxide | | 1.4 | 1425.3 | 1424.0 | - | 7.8 |
| Nitrogen | | - | 38.4 | 38.4 | 6.5 | 1374.0 |
| Argon | | - | 20.6 | 20.6 | 3.5 | 20.9 |
| Methane | | - | 13.7 | 13.7 | 2.3 | - |
| Ethane | | - | - | - | - | - |
| Propane | | - | - | - | - | - |
| Butane | | - | - | - | - | - |
| Pentane + | | - | - | - | - | - |
| Methanol | | - | - | - | - | - |
| Oxygen | | - | - | - | - | 67.2 |
| Lights | | - | - | - | - | - |
| Heavies | | - | - | - | - | - |

### Example 2 - Comparative

The same flowsheet as Example 1 was modelled except without the purge gas treatment unit 78 and with the carbon rich off gas stream 74 and natural gas used as fuel in the fired steam reformer 20. The differences in emissions from the process comparing the fired reformer flue gas 24 of Example 2 versus the fired reformer flue gas 24 and fired heater flue gas 114 of Example 1 are as follows:

| Stream Number | | Comparative Example 2 | Example 1 |
|---|---|---|---|
| Temperature | °C | 140 | 150 |
| Pressure | bar a | 1.02 | 1.02 |
| Mass Flow | tonne/h | 366.7 | 291.0 |
| Molar Flow | kgmole/h | 13482 | 11661 |
| Molecular Weight | | 27.20 | 24.95 |
| Composition | kgmole/h | | |
| Water | | 3199.2 | 3859 |
| Carbon Dioxide | | 1181.0 | 46.4 |
| Nitrogen | | 8798.1 | 7429.6 |
| Argon | | 109.2 | 115.1 |
| Oxygen | | 192.8 | 210.7 |

The invention therefore provides a total CO₂ reduction of 1134.6 kmol/h or 49.9t/h or 416,000tpa (base on 8333h/pa). This corresponds to a 96.1% reduction in CO₂ emissions with a 96.8% CO₂ capture.

## Claims

1. A process for synthesising methanol comprising the steps of (i) reforming a hydrocarbon feedstock in a hydrocarbon reforming unit comprising a fired steam reformer to form a synthesis gas containing hydrogen, carbon monoxide and carbon dioxide; (ii) converting the synthesis gas into a methanol product in a methanol loop comprising one or more methanol synthesis reactors; and (iii) recovering a purge gas stream from the methanol loop, wherein at least a portion of the purge gas stream is treated in a purge gas treatment unit by subjecting it to partial oxidation in a partial oxidation reactor or autothermal reforming in a purge gas reforming unit to form a partially-oxidised or reformed purge gas, followed by one or more stages of water gas shift of the partially-oxidised or reformed purge gas in a water-gas shift unit to form a hydrogen-enriched gas, and a step of carbon dioxide removal from the hydrogen-enriched gas in a carbon dioxide removal unit to form a hydrogen stream and a carbon dioxide stream, wherein the carbon dioxide stream is recovered and a portion of the hydrogen stream is fed to the fired steam reformer as a fuel.

2. A process according to claim 1, wherein the hydrocarbon reforming unit comprises a fired steam reformer and an autothermal reformer and the process comprises feeding an oxygen containing gas and a reformed gas from the fired steam reformer to the autothermal reformer to generate the synthesis gas.

3. A process according to claim 2, wherein the reformed gas from the fired steam reformer is mixed with a portion of the hydrocarbon feedstock.

4. A process according to claim 2 or claim 3, wherein a hydrogen-rich gas stream is added to the synthesis gas upstream of the methanol loop.

5. A process according to any one of claims 1 to 4, wherein one or more feeds to the purge gas treatment unit is heated using a fired heater that is fired by a portion of the hydrogen stream.

6. A process according to any one of claims 1 to 5, wherein the one or more stages of water gas shift comprises an isothermal shift reactor cooled by boiling water under pressure.

7. A process according to any one of claims 1 to 6, wherein at least a portion of the carbon dioxide recovered from the carbon dioxide removal unit is fed to the synthesis gas upstream of the one or more methanol synthesis reactors.

8. A process according to any one of claims 1 to 7, wherein at least a portion of the purge gas is fed directly to the purge gas treatment unit.

9. A process according to any one of claims 1 to 8, wherein a hydrogen-rich gas stream is separated from the purge gas upstream of the purge gas treatment unit thereby forming a carbon-rich purge gas which is fed to the purge gas treatment unit.

10. A method for retrofitting a methanol production unit comprising a hydrocarbon reforming unit comprising a fired steam reformer and a methanol loop comprising one or more methanol synthesis reactors, wherein the methanol loop is fed with a synthesis gas from the hydrocarbon reforming unit and generates a methanol product and a purge gas stream, by (i) installing a purge gas treatment unit comprising a partial oxidation reactor or a purge gas reforming unit, a water-gas shift unit, a carbon dioxide removal unit and means to feed a hydrogen stream recovered from the carbon dioxide removal unit to the fired steam reformer as a fuel, (ii) passing at least a portion of the purge gas stream to the partial oxidation reactor or the purge gas reforming unit to form a partially-oxidised or reformed purge gas stream, (iii) subjecting the partially-oxidised or reformed purge gas stream to one or more stages of water gas shift in the water-gas shift unit to form a hydrogen-enriched stream, (iv) subjecting the hydrogen-enriched stream to carbon dioxide removal in the carbon dioxide removal unit to form a hydrogen stream, and (v) feeding the hydrogen stream to the fired steam reformer as a fuel.

11. A method according to claim 10, further comprising installation of H₂ fuel burners in the fired steam reformer.

12. A method according to claim 10 or claim 11, wherein the hydrocarbon reforming unit comprises a fired steam reformer fed with a portion of a hydrocarbon feed and an autothermal reformer fed with a further portion of the hydrocarbon feed and a reformed gas from the fired steam reformer, wherein hydrogen is recovered from the purge gas stream upstream of the purge gas treatment unit and fed to the methanol loop, and a carbon-rich purge gas generated by recovery of the hydrogen from the purge gas stream is fed to the purge gas treatment unit.

13. A method according to claim 10 or claim 11, wherein the hydrocarbon reforming unit consists of a fired steam reformer and the partial oxidation reactor or autothermal reformer in the purge gas treatment unit uses a pure oxygen gas, further comprising recovering a portion of the partially oxidised or reformed gas upstream of the water-gas shift unit in the purge gas treatment unit and adding it to the synthesis gas upstream of the methanol loop, or to a synthesis gas fed to one or more methanol synthesis reactors in a neighbouring methanol production unit.

14. A method according to any one of claims 10 to 13, wherein carbon dioxide recovered from the carbon dioxide removal unit is fed to the synthesis gas upstream of the one or more methanol synthesis reactors.

15. A method according to claim 14, wherein after installation of the purge gas treatment unit the hydrocarbon reforming unit is operated at a lower outlet temperature.

## Patentansprüche

1. Verfahren zur Synthese von Methanol, umfassend die Schritte (i) Reformieren eines Kohlenwasserstoff-Ausgangsmaterials in einer Kohlenwasserstoffreformierungseinheit, umfassend einen befeuerten Dampfreformer, um ein Synthesegas zu bilden, das Wasserstoff, Kohlenmonoxid und Kohlendioxid enthält; (ii) Umwandeln des Synthesegases in ein Methanolprodukt in einem Methanolkreislauf, umfassend einen oder mehrere Methanolsynthesereaktoren; und (iii) Rückgewinnen eines Spülgasstroms aus dem Methanolkreislauf, wobei mindestens ein Teil des Spülgasstroms in einer Spülgasbehandlungseinheit durch Unterziehen einer partiellen Oxidation in einem Partialoxidationsreaktor oder einer autothermen Reformierung in einer Spülgasreformierungseinheit behandelt wird, um ein partiell oxidiertes oder reformiertes Spülgas zu bilden, gefolgt von einer oder mehreren Stufen eines Wassergas-Shifts des partiell oxidierten oder reformierten Spülgases in einer Wassergas-Shift-Einheit, um ein wasserstoffangereichertes Gas zu bilden, und einem Schritt der Kohlendioxidentfernung aus dem wasserstoffangereicherten Gas in einer Kohlendioxidentfernungseinheit, um einen Wasserstoffstrom und einen Kohlendioxidstrom zu bilden, wobei der Kohlendioxidstrom zurückgewonnen wird und dem befeuerten Dampfreformer ein Teil des Wasserstoffstroms als Brennstoff zugeführt wird.

2. Verfahren nach Anspruch 1, wobei die Kohlenwasserstoffreformierungseinheit einen befeuerten Dampfreformer und einen autothermen Reformer umfasst und das Verfahren das Zuführen eines sauerstoffhaltigen Gases und eines reformierten Gases aus dem befeuerten Dampfreformer zu dem autothermen Reformer umfasst, um das Synthesegas zu erzeugen.

3. Verfahren nach Anspruch 2, wobei das reformierte Gas aus dem befeuerten Dampfreformer mit einem Teil des Kohlenwasserstoff-Ausgangsmaterials gemischt wird.

4. Verfahren nach Anspruch 2 oder 3, wobei dem Synthesegas stromaufwärts des Methanolkreislaufs ein wasserstoffreicher Gasstrom zugesetzt wird.

5. Verfahren nach einem der Ansprüche 1 bis 4, wobei eine oder mehrere Zufuhren zur Spülgasbehandlungseinheit unter Verwendung einer befeuerten Heizvorrichtung erhitzt werden, die mit einem Teil des Wasserstoffstroms befeuert wird.

6. Verfahren nach einem der Ansprüche 1 bis 5, wobei die eine oder mehreren Stufen des Wassergas-Shifts einen isothermen Shift-Reaktor umfassen, der durch unter Druck stehendes siedendes Wasser gekühlt wird.

7. Verfahren nach einem der Ansprüche 1 bis 6, wobei mindestens ein Teil des Kohlendioxids, das aus der Kohlendioxidentfernungseinheit gewonnen wird, dem Synthesegas stromaufwärts des einen oder der mehreren Methanolsynthesereaktoren zugeführt wird.

8. Verfahren nach einem der Ansprüche 1 bis 7, wobei mindestens ein Teil des Spülgases direkt der Spülgasbehandlungseinheit zugeführt wird.

9. Verfahren nach einem der Ansprüche 1 bis 8, wobei ein wasserstoffreicher Gasstrom stromaufwärts der Spülgasbehandlungseinheit von dem Spülgas getrennt wird, wodurch ein kohlenstoffreiches Spülgas gebildet wird, das der Spülgasbehandlungseinheit zugeführt wird.

10. Verfahren zum Nachrüsten einer Methanolproduktionseinheit, umfassend eine Kohlenwasserstoffreformierungseinheit, umfassend einen befeuerten Dampfreformer und einen Methanolkreislauf, umfassend einen oder mehrere Methanolsynthesereaktoren, wobei der Methanolkreislauf mit einem Synthesegas aus der Kohlenwasserstoffreformierungseinheit gespeist wird und ein Methanolprodukt und einen Spülgasstrom erzeugt, durch (i) Installieren einer Spülgasbehandlungseinheit, umfassend einen Partialoxidationsreaktor oder eine Spülgasreformierungseinheit, eine Wassergas-Shift-Einheit, eine Kohlendioxidentfernungseinheit und Mittel zum Zuführen eines aus der Kohlendioxidentfernungseinheit gewonnenen Wasserstoffstroms zum befeuerten Dampfreformer als Brennstoff, (ii) Leiten mindestens eines Teils des Spülgasstroms zum Partialoxidationsreaktor oder zur Spülgasreformierungseinheit, um einen partiell oxidierten oder reformierten Spülgasstrom zu bilden, (iii) Unterziehen des partiell oxidierten oder reformierten Spülgasstroms einer oder mehreren Stufen des Wassergas-Shifts in der Wassergas-Shift-Einheit, um einen wasserstoffangereicherten Strom zu bilden, (iv) Unterziehen des wasserstoffangereicherten Stroms einer Kohlendioxidentfernung in der Kohlendioxidentfernungseinheit, um einen Wasserstoffstrom zu bilden, und (v) Zuführen des Wasserstoffstroms zu dem befeuerten Dampfreformer als Brennstoff.

11. Verfahren nach Anspruch 10, ferner umfassend die Installation von H₂-Brennstoffbrennern in dem befeuerten Dampfreformer.

12. Verfahren nach Anspruch 10 oder 11, wobei die Kohlenwasserstoffreformierungseinheit einen befeuerten Dampfreformer, der mit einem Teil einer Kohlenwasserstoffzufuhr gespeist wird, und einen autothermen Reformer, der mit einem weiteren Teil der Kohlenwasserstoffzufuhr und einem reformierten Gas aus dem befeuerten Dampfreformer gespeist wird, umfasst, wobei Wasserstoff aus dem Spülgasstrom stromaufwärts der Spülgasbehandlungseinheit zurückgewonnen und dem Methanolkreislauf zugeführt wird, und ein kohlenstoffreiches Spülgas, das durch Rückgewinnung des Wasserstoffs aus dem Spülgasstrom erzeugt wird, der Spülgasbehandlungseinheit zugeführt wird.

13. Verfahren nach Anspruch 10 oder 11, wobei die Kohlenwasserstoffreformierungseinheit aus einem befeuerten Dampfreformer besteht und der Partialoxidationsreaktor oder autotherme Reformer in der Spülgasbehandlungseinheit ein reines Sauerstoffgas verwendet, ferner umfassend das Rückgewinnen eines Teils des partiell oxidierten oder reformierten Gases stromaufwärts der Wassergas-Shift-Einheit in der Spülgasbehandlungseinheit und das Zusetzen dessen dem Synthesegas stromaufwärts des Methanolkreislaufs oder einem Synthesegas, das einem oder mehreren Methanolsynthesereaktoren in einer benachbarten Methanolproduktionseinheit zugeführt wird.

14. Verfahren nach einem der Ansprüche 10 bis 13, wobei Kohlendioxid, das aus der Kohlendioxidentfernungseinheit gewonnen wird, dem Synthesegas stromaufwärts des einen oder den mehreren Methanolsynthesereaktoren zugeführt wird.

15. Verfahren nach Anspruch 14, wobei die Kohlenwasserstoffreformierungseinheit nach der Installation der Spülgasbehandlungseinheit bei einer niedrigeren Auslasstemperatur betrieben wird.

## Revendications

1. Processus permettant de synthétiser du méthanol comprenant les étapes consistant à (i) reformer un produit de départ hydrocarboné dans une unité de reformage d'hydrocarbure comprenant un reformeur à vapeur à combustion pour former un gaz de synthèse contenant de l'hydrogène, du monoxyde de carbone et du dioxyde de carbone ; (ii) convertir le gaz de synthèse en un produit de méthanol dans une boucle de méthanol comprenant un ou plusieurs réacteurs de synthèse de méthanol ; et (iii) récupérer un courant de gaz de purge provenant de la boucle de méthanol, dans lequel au moins une partie du courant de gaz de purge est traitée dans une unité de traitement de gaz de purge en la soumettant à une oxydation partielle dans un réacteur d'oxydation partielle ou à un reformage autothermique dans une unité de reformage de gaz de purge pour former un gaz de purge partiellement oxydé ou reformé, suivi par une ou plusieurs phases de conversion eau-gaz du gaz de purge partiellement oxydé ou reformé dans une unité de conversion eau-gaz pour former un gaz enrichi en hydrogène, et une étape d'élimination de dioxyde de carbone du gaz enrichi en hydrogène dans une unité d'élimination de dioxyde de carbone pour former un courant d'hydrogène et un courant de dioxyde de carbone, dans lequel le courant de dioxyde de carbone est récupéré et une partie du courant d'hydrogène est alimentée au reformeur à vapeur à combustion en tant que combustible.

2. Processus selon la revendication 1, dans lequel l'unité de reformage d'hydrocarbure comprend un reformeur à vapeur à combustion et un reformeur autothermique et le processus comprend l'alimentation d'un gaz contenant de l'oxygène et d'un gaz reformé provenant du reformeur à vapeur à combustion au reformeur autothermique pour générer le gaz de synthèse.

3. Processus selon la revendication 2, dans lequel le gaz reformé provenant du reformeur à vapeur à combustion est mélangé avec une partie du produit de départ hydrocarboné.

4. Processus selon la revendication 2 ou la revendication 3, dans lequel un courant de gaz riche en hydrogène est ajouté au gaz de synthèse en amont de la boucle de méthanol.

5. Processus selon l'une quelconque des revendications 1 à 4, dans lequel une ou plusieurs alimentations vers l'unité de traitement de gaz de purge sont chauffées à l'aide d'un dispositif de chauffage à combustion dont la combustion est alimentée par une partie du courant d'hydrogène.

6. Processus selon l'une quelconque des revendications 1 à 5, dans lequel la ou les phases de conversion eau-gaz comprennent un réacteur de conversion isotherme refroidi par de l'eau bouillante sous pression.

7. Processus selon l'une quelconque des revendications 1 à 6, dans lequel au moins une partie du dioxyde de carbone récupéré de l'unité d'élimination de dioxyde de carbone est alimentée au gaz de synthèse en amont du ou des réacteurs de synthèse de méthanol.

8. Processus selon l'une quelconque des revendications 1 à 7, dans lequel au moins une partie du gaz de purge est alimentée directement à l'unité de traitement de gaz de purge.

9. Processus selon l'une quelconque des revendications 1 à 8, dans lequel un courant de gaz riche en hydrogène est séparé du gaz de purge en amont de l'unité de traitement de gaz de purge en formant de ce fait un gaz de purge riche en carbone qui est alimenté à l'unité de traitement de gaz de purge.

10. Procédé permettant de modifier ultérieurement une unité de production de méthanol comprenant une unité de reformage d'hydrocarbure comprenant un reformeur à vapeur à combustion et une boucle de méthanol comprenant un ou plusieurs réacteurs de synthèse de méthanol, dans lequel la boucle de méthanol est alimentée avec un gaz de synthèse provenant de l'unité de reformage d'hydrocarbure et génère un produit de méthanol et un courant de gaz de purge, par (i) l'installation d'une unité de traitement de gaz de purge comprenant un réacteur d'oxydation partielle ou une unité de reformage de gaz de purge, une unité de conversion eau-gaz, une unité d'élimination de dioxyde de carbone et un moyen pour alimenter un courant d'hydrogène récupéré de l'unité d'élimination de dioxyde de carbone au reformeur à vapeur à combustion en tant que combustible, (ii) le passage d'au moins une partie du courant de gaz de purge vers le réacteur d'oxydation partielle ou l'unité de reformage de gaz de purge pour former un courant de gaz de purge partiellement oxydé ou reformé, (iii) le fait de soumettre le courant de gaz de purge partiellement oxydé ou reformé à une ou plusieurs phases de conversion eau-gaz dans l'unité de conversion eau-gaz pour former un courant enrichi en hydrogène, (iv) le fait de soumettre le courant enrichi en hydrogène à une élimination de dioxyde de carbone dans l'unité d'élimination de dioxyde de carbone pour former un courant d'hydrogène, et (v) l'alimentation du courant d'hydrogène au reformeur à vapeur à combustion en tant que combustible.

11. Procédé selon la revendication 10, comprenant en outre l'installation de brûleurs à combustible H₂ dans le reformeur à vapeur à combustion.

12. Procédé selon la revendication 10 ou la revendication 11, dans lequel l'unité de reformage d'hydrocarbure comprend un reformeur à vapeur à combustion alimenté avec une partie d'une alimentation d'hydrocarbures et un reformeur autothermique alimenté avec une partie supplémentaire de l'alimentation d'hydrocarbures et un gaz reformé provenant du reformeur à vapeur à combustion, dans lequel de l'hydrogène est récupéré du courant de gaz de purge en amont de l'unité de traitement de gaz de purge et alimenté à la boucle de méthanol, et un gaz de purge riche en carbone généré par récupération de l'hydrogène provenant du courant de gaz de purge est alimenté à l'unité de traitement de gaz de purge.

13. Procédé selon la revendication 10 ou la revendication 11, dans lequel l'unité de reformage d'hydrocarbure est constituée d'un reformeur à vapeur à combustion et le réacteur d'oxydation partielle ou le reformeur autothermique dans l'unité de traitement de gaz de purge utilise de l'oxygène gazeux pur, comprenant en outre la récupération d'une partie du gaz partiellement oxydé ou reformé en amont de l'unité de conversion eau-gaz dans l'unité de traitement de gaz de purge et son ajout au gaz de synthèse en amont de la boucle de méthanol, ou à un gaz de synthèse alimenté à un ou plusieurs réacteurs de synthèse de méthanol dans une unité de production de méthanol voisine.

14. Procédé selon l'une quelconque des revendications 10 à 13, dans lequel du dioxyde de carbone récupéré de l'unité d'élimination de dioxyde de carbone est alimenté au gaz de synthèse en amont du ou des réacteurs de synthèse de méthanol.

15. Procédé selon la revendication 14, dans lequel après installation de l'unité de traitement de gaz de purge l'unité de reformage d'hydrocarbure est exploitée à une température de sortie plus basse.
